# EUROPÄISCHE PATENTSCHRIFT

(11) **EP 1 774 911 B1**
(45) Veröffentlichungstag und Bekanntmachung des Hinweises auf die Patenterteilung: **04.11.2009**
(21) Anmeldenummer: 06121313.8
(22) Anmeldetag: 27.09.2006
(51) Int. Cl.: A61B 6/10, A61B 6/00

(54) **Medizinisches Bildgebungssystem und Kollisionsschutzverfahren**
Medical imaging system and method for protection against collision
Système d'imagerie médicale et procédé d'arbitrage de collision

(30) Priorität: 13.10.2005 DE 102005049106
(43) Veröffentlichungstag der Anmeldung: 18.04.2007
(73) Patentinhaber: SIEMENS AKTIENGESELLSCHAFT, 80333 München (DE)
(72) Erfinder: Spahn, Martin Dr., Chicago, IL 60622 (US)

(56) Entgegenhaltungen:
- DE-A1- 19 743 500
- DE-A1- 19 855 213
- DE-A1-102004 042 790
- DE-A1-102005 023 165
- DE-C1- 4 335 301
- US-A- 5 654 997

## Beschreibung

Die Erfindung betrifft ein medizinisches Bildgebungssystem mit einem um einen Patienten verfahrbaren Teil und insbesondere ein System, bei welchem die Bewegung des verfahrbaren Teils durch ein Regelgerät geregelt wird, welches die Bewegung stoppt oder verlangsamt, wenn sich das Teil dem Patienten nähert, d.h. einen vorbestimmbaren Mindestabstand zu bestimmten Oberflächenbereichen des Patienten unterschreitet oder sich diesen nähert.

Im Stand der Technik sind medizinische Bildgebungssysteme, insbesondere Röntgensysteme bekannt, die sich durch eine große Flexibilität in den Bewegungen der Bildaufnahmegeräte um den Patienten auszeichnen. Im Falle eines Röntgensystems handelt es sich bei diesen verfahrbaren Teilen insbesondere um den Röntgendetektor und die Röntgenröhre. Großer Beliebtheit erfreuen sich insbesondere die so genannten C-Bogen-Röntgensysteme, bei welchen Röntgenröhre- und Detektor jeweils an einander gegenüberliegenden Armen eines C-Bogens befestigt sind, welcher beliebig um den Patienten verfahrbar ist, um so Röntgenaufnahmen aus beliebigen Projektionsrichtungen zu ermöglichen. Durch Variation des Abstandes zwischen Röntgendetektor und Patient kann darüber hinaus die Vergrößerung verändert und die bildverrauschende Streustrahlung minimiert werden. Solche Systeme, wie z. B. das AXIOM Artis der Siemens AG, werden insbesondere als Angiographiesysteme benutzt. Zunehmend werden mit solchen Röntgengeräten auch quasi-tomographische 3D-Bilder erzeugt, bei denen der C-Bogen um ca. 180° um den Patienten verfahren wird. Bei einem solchen Durchlauf, der auch mit "DynaRun" bezeichnet wird, werden die beweglichen Teile zum Teil mit erheblicher Geschwindigkeit um den Patienten rotiert. Ein Problem derartiger Bildgebungssysteme mit verfahrbaren Teilen liegt darin, dass eine Kollision der verfahrbaren Teile mit dem Patienten möglich ist. Um Gefährdungen für den Patienten auszuschließen, müssen daher Schutzmechanismen eingeführt werden.

Digitale Bildgebungsverfahren spielen inzwischen eine entscheidende Rolle in der medizinischen Diagnostik. Während in diagnostischen Verfahren, wie zum Beispiel der Computertomographie, der Magnetresonanz, dem Ultraschall und Verfahren der Nuklearmedizin digitale Techniken von Beginn an eingesetzt wurden, findet derzeit insbesondere mit Hilfe der Flachdetektoren in "konventionellen" Röntgenverfahren, wie der Radiographie, der Mammographie, der Angiographie oder auch der Kardiologie der Übergang zur digitalen Bildgebung in hohem Maße statt (siehe beispielsweise Spahn et al. "Digitale Röntgen-Detektoren in der Röntgendiagnostik", Radiologie 43 (2003), Seiten 340 bis 350).

Diese neuen Verfahren erfordern immer schnellere Akquisitionen bzw. eine immer größere Anzahl von Bildern für eine dreidimensionale Bild-Akquisition, wobei beispielsweise bei einer gewöhnlichen Projektions-Radiographie inzwischen auch Roboterarme eingesetzt werden, die durch die Anwahl eines Organprogramms angesteuert werden können und selbstständig in eine neue Position verfahren. Auch hier besteht ein Interesse, das Verfahren möglichst schnell durchzuführen, um den Ablauf im Krankenhaus zu beschleunigen.

Auch hier besteht die Gefahr, dass eine Kollision des verfahrbaren Teils, wie z.B. des Roboterarms, mit dem Patienten möglich ist. Insbesondere bei schnellen Bewegungen von mechanischen Tragarmen besteht grundsätzlich die Gefahr einer Kollision zwischen diesem Tragarm und dem Patienten oder dem Tragarm und einer anderen mechanischen Komponente, wie beispielsweise dem Tisch, einem zweiten Tragarm oder anderen medizinischen Geräten. Dabei sind verschiedene Vorrichtungen und Verfahren bekannt, um eine Kollision zu vermeiden oder um die Auswirkung einer Kollision abzuschwächen:

Bei dem oben genannten System AXIOM Artis wird z. B. eine den Patienten umhüllende Schutzzone definiert. Nähert sich ein C-Arm an diese Schutzzone an, wird er deutlich verlangsamt, um die Gefahr einer Kollision zu vermeiden. Diese Schutzzone ist für alle Patienten gleich und hat angenähert die Form eines über der Patientenliege angeordneten Ellipsoids. Insbesondere bei dünnen Patienten liegt diese Schutzzone daher oft weit entfernt von der tatsächlichen Patientenoberfläche. Daher dauert es oft unnötig lange, um eine bestimmte Angulation des C-Bogens anzufahren.

Bei den Geräten der Firma Philips befinden sich teilweise an den C-Armen kapazitive Sensoren, welche die Nähe des Patienten detektieren und daraufhin die Bewegung des C-Arms verlangsamen. Diese Sensoren haben jedoch nur eine geringe Reichweite, so dass auch hier die Bewegung des C-Bogens bei Eintritt in eine angenommene Schutzzone verlangsamt werden sollte, da es sonst bei schnellen Bewegungen zur Kollision kommt.

Als letzte Sicherheit weisen beide Systeme mechanische Rückmelder auf, die bei einem tatsächlichen Patientenkontakt sofort die Bewegung der verfahrbaren Teile stoppen (vgl. auch EP 0 395 352 A1, die bei Kollision eine Entkopplung des Arm-Motors von der Stromversorgung offenbart).

Ein Nachteil der mechanischen Ausführung eines Kollisionsschutzes oder der Berechnung einer Schutzzone ist es, dass das Verfahren entweder auf Kontakt beruht oder umfangreiche Rechenarbeiten erfordert, so dass bei den zunehmend schneller werdenden Bewegungen des verfahrbaren Teils die Reaktionszeit, die dadurch immer kürzer wird, oft nicht ausreicht, um eine Kollision tatsächlich zu verhindern, bzw. es werden dann zunehmend höhere Bremsimpulse nötig, um das verfahrbare Teil abzufangen. Dies erfordert wiederum stabilere Mechaniken und leistungsfähigere Motoren bzw. Bremssysteme, was wiederum die Kosten und die Masse der Systeme erhöht, wodurch letztendlich erneut die Bremsimpulse und der Bewegungsablauf des verfahrbaren Teils negativ beeinflusst wird.

Ein medizinisches Bildgebungssystem mit den Merkmalen des ersten Teils von Anspruch 1 ist aus DE-A-198 55 213 bekannt. Hieraus folgt - mutatis mutandis- ein Kollisionsschutzverfahen.

Die Erfindung hat sich die Aufgabe gestellt, ein medizinisches Bildgebungssystem mit einem schnelleren, genaueren und einfacheren Kollisionsschutzsystem, sowie ein entsprechendes Kollisionsschutzverfahren, bereit zu stellen.

Diese Aufgabe löst die Erfindung mit den kennzeichnenden Merkmalen der Ansprüche 1 und 10. Bevorzugte Ausführungsformen des erfindungsgemäßen Bildgebungssystems und Kollisionsschutzverfahren sind in den jeweils abhängigen Ansprüchen angegeben.

Erfindungsgemäß wird das medizinische Bildgebungssystem, das ein relativ zum Patienten verfahrbares Teil, wie beispielsweise einen C-Bogen oder einen Roboterarm aufweist, mit mindestens einem Sensor ausgestattet, der die Oberfläche des Patienten oder eines anderen Objekts erfasst, während ein Regelgerät, das die Bewegung des verfahrbaren Teils regelt, auf Grund der berechneten Abstände zwischen dem verfahrbaren Teil und der Oberfläche des Patienten oder eines anderen Objekts entsprechend die Bewegung des verfahrbaren Teils regelt, das heißt, beispielsweise stoppt oder verlangsamt, wenn sich das Teil dem Patienten oder einem anderen Objekt nähert bzw. diese berührt.

Das Regelgerät berechnet aus der erfassten Oberfläche des Patienten die Abstände des verfahrbaren Teils zur Oberfläche des Patienten oder eines anderen Objekts, das im Verfahrweg liegt und regelt entsprechend die Bewegung. Der mindestens eine Sensor übermittelt hierfür die Positionen einzelner Oberflächenbereiche des Patienten oder eines anderen Objekts an das Regelgerät, gleichzeitig erhält das Regelgerät die Position des verfahrbaren Teils von einem zweiten Sensor, der die Position des verfahrbaren Teils detektiert und an das Regelgerät übermittelt. Im Anschluss daran kann das Regelgerät aus den Positionen die Abstände zwischen dem verfahrbaren Teil und entsprechenden Oberflächenbereichen ermitteln und die Geschwindigkeit bzw. die Verfahrrichtung des verfahrbaren Teils entsprechend regeln.

Der Sensor zur Ermittlung der Oberflächenbereiche kann ein Ultraschall-Sensor, ein optischer Sensor oder ein elektromagnetischer Sensor sein. Insbesondere werden keine auf Kontakt basierenden Sensoren, wie im Stand der Technik eingesetzt, sondern Sensoren, die bereits auf Distanz eine Annäherung zwischen einer Sensor-bestückten Systemkomponente, wie beispielsweise dem verfahrbaren Teil und externen Objekten, wie dem Patienten orten und angeben können. Solche Sensoren sind für besonders schnelle Bewegungen, wie beispielsweise bei der 3D-Akquisition, vorteilhaft.

Erfindungsgemäß sind die Sensoren im Detektorgehäuse des Bildgebungsgeräts oder in bzw. an der Röntgenröhre oder anderen exponierten Bereichen des verfahrbaren Teils integriert.

Der zweite Sensor kann - nach einer weiteren vorteilhaften Ausführungsform der Erfindung - ebenfalls ein elektromagnetischer Sensor sein, der aus einem ersten Sensorteil besteht, das ein elektromagnetisches Feld im Raum um den Patienten bzw. um das medizinische Bildgebungssystem herum generiert und aus einem zweiten Sensorteil, das am verfahrbaren Teil angebracht ist und die Position des verfahrbaren Teils im elektromagnetischen Feld detektiert. Wahlweise kann der zweite Sensor aber auch ein optischer, ein Beschleunigungs- oder ein Ultraschall-Sensor sein, der die Position des verfahrbaren Teils detektiert.

Mit Vorteil ist das Bildgebungssystem ein Röntgensystem, und das verfahrbare Teil ist ein Röntgendetektor oder eine Röntgenröhre. Insbesondere ist das Bildgebungssystem ein C-Röntgenbogen-System, bei welchem der Röntgendetektor und die Röntgenröhre an einem großen C-Bogen befestigt sind, der um den Patienten verfahrbar ist. Dabei werden nach einer weiteren vorteilhaften Ausführungsform der Erfindung mehrere (Kollisions-)Sensoren an verschiedenen Stellen des verfahrbaren Teils befestigt.

Mit Vorteil ist das Koordinatensystem des Bildgebungssystems und das des Sensors oder der Sensoren räumlich zueinander kalibriert. Dies erleichtert dem Regelgerät die Berechnung der Abstände zwischen dem verfahrbaren Teil und den Oberflächenbereichen des externen Objekts, wie beispielsweise der Oberfläche des Patienten. Darüber hinaus kann das Bildgebungssystem auch dazu ausgelegt sein, aus der erfassten Oberfläche des Patienten ein 3D-Modell des Patienten zu erstellen und dieses mit dem Koordinatensystem des Bildgebungssystems zu registrieren. In diesem Fall kann das Regelgerät die Bewegung des verfahrbaren Teils exakt vorherbestimmen. Mit Vorteil wird dieses 3D-Modell in regelmäßigen Abständen aktualisiert, um zu vermeiden, dass bei Bewegungen des Patienten den Vorausberechnungen des Regelgeräts ein falsches 3D-Modell zugrunde liegt. Alle Berechnungen können auch von einer externen Recheneinheit durchgeführt werden, die die Ergebnisse ans Regelgerät liefert.

Die vorliegende Erfindung betrifft darüber hinaus ein Kollisionsschutzverfahren für ein medizinisches Bildgebungssystem, mit einem um einen Patienten verfahrbaren Teil, zur Verhinderung einer Kollision des verfahrbaren Teils mit einem externen Objekt, wie beispielsweise dem Patienten, wobei die Bewegung des verfahrbaren Teils in Abhängigkeit vom Abstand zum Patienten durch ein Regelgerät regelbar ist, indem die Oberfläche des Patienten durch mindestens einen Sensor erfasst wird und das Regelgerät auf Grund der berechneten Abstände zwischen dem verfahrbaren Teil und der Oberfläche des Objekts entsprechend die Bewegung des verfahrbaren Teils regelt.

In dem Verfahren werden die Positionen einzelner Oberflächenbereiche des Objekts durch den mindestens einen Sensor detektiert und an das Regelgerät übermittelt. Die Positionen des verfahrbaren Teils werden fortlaufend mittels eines zweiten Sensors detektiert und an das Regelgerät weitergeleitet. Das Regelgerät kann dann die Abstände zwischen dem verfahrbaren Teil und den entsprechenden Oberflächenbereichen des Patienten bzw. eines anderen Objekts ermitteln und die Geschwindigkeit bzw. die Verfahrrichtung des verfahrbaren Teils entsprechend regeln. Nach einer bevorzugten Ausführungsform der Erfindung wird die Erfassung der Oberfläche durch einen optischen Sensor ermöglicht, wobei des Koordinatensystem des Bildgebungssystems und das des optischen Sensors vor der Aufnahme von entsprechenden Bilddaten zueinander kalibriert werden.

Eine vorteilhafte Ausführungsform der Erfindung wird an Hand der beiliegenden Zeichnungen näher erläutert. Dabei zeigen:
- Fig. 1: ein medizinisches Bildgebungssystem mit Kollisionssensoren an exponierten Stellen der verfahrbaren Teile,
- Fig. 2: ein Röntgen-C-Bogen-System mit entsprechenden Kollisionssensoren , und
- Fig. 3: ein Roboterarm-System nach der vorliegenden Erfindung mit entsprechenden Kollisionssensoren an exponierten Stellen der verfahrbaren Arme.

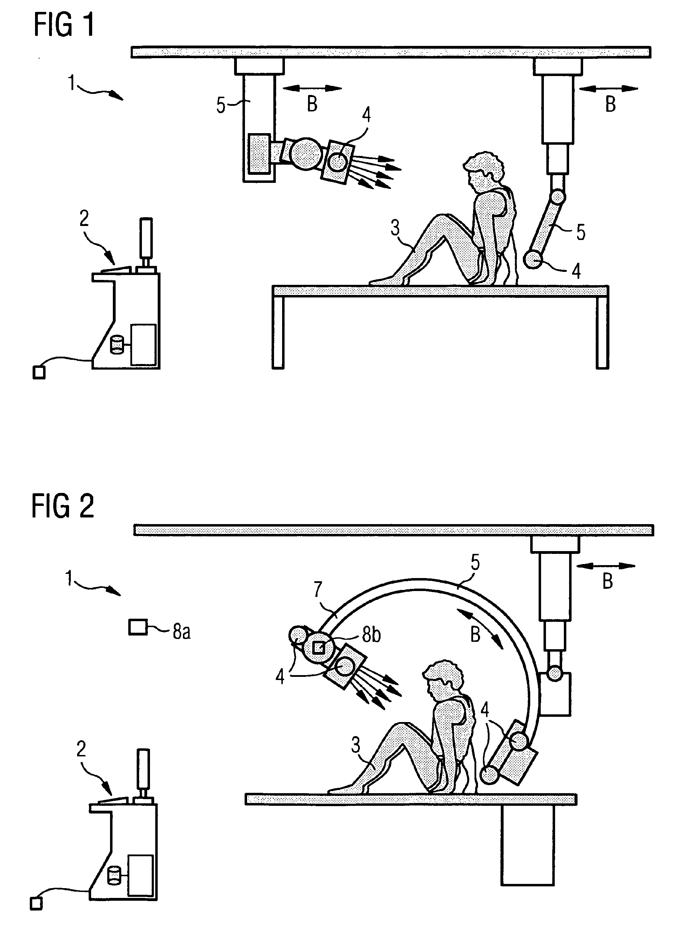

Fig. 1 zeigt ein medizinisches Bildgebungssystem 1 mit einem schematisch dargestellten Patienten 3, der auf einem Tisch sitzt. Ein 3D-Stativ für den Röntgenstrahler (links im Bild dargestellt) und ein 3D-Stativ für den Röntgendetektor werden auf beiden Seiten des Patienten positioniert, sodass beispielsweise Durchleuchtungsaufnahmen der Lunge des Patienten angefertigt werden können. Zur Justierung können beide Stative in der Bewegungsrichtung B oder senkrecht dazu verfahren werden. Darüber hinaus kann der Röntgendetektor gedreht werden. Dieses verfahrbare Teil 5 wird mit einem Sensor 4 versehen, ebenso wie der Röntgenstrahler auch mit einem Sensor 4 ausgestattet wird. Durch die zwei Sensoren 4 können entsprechende Abstände zwischen den verfahrbaren Teilen 5 und der Oberfläche des Patienten 3 detektiert und an ein Regelgerät 2 übertragen werden, das die Bewegung B der verfahrbaren Teile 5 regelt.

Fig. 2 zeigt ein Röntgen-C-Bogen-System, beispielsweise für die Kardiologie oder auch Angiographie, mit Sensoren 4 an entsprechend exponierten Stellen des Röntgenstrahlers und des Röntgendetektors. Der C-Bogen kann ebenfalls in Bewegungsrichtung B bewegt werden, wie der C-Bogen um den Patienten 3 rotieren kann. Das verfahrbare Teil 5 ist in diesem Fall der C-Bogen 7, der um den Patienten 3 rotiert. Auch hier werden die Abstandsdaten der Sensoren 4 an ein Regelgerät 2 übertragen.

Nach der Erfindung detektiert ein zweiter Sensor 8 die jeweilige Position des C-Bogens 7, indem ein zweiter Teil 8b des zweiten Sensors 8 am Röntgenstrahler angebracht wird und so entsprechende Positionsdaten an den ersten Teil 8a des zweiten Sensors 8 oder direkt an das Regelgerät 2 übermittelt. Derartige Positionsdetektoren sind im Stand der Technik bekannt, beispielsweise eignet sich hier ein elektromagnetisches System, wobei der erste Sensorteil 8a ein elektromagnetisches Feld aufbaut, das im zweiten Sensorteil 8b bei Bewegung des verfahrbaren Teils 5 entsprechend detektiert wird, so dass Positionsänderungen festgestellt werden können.

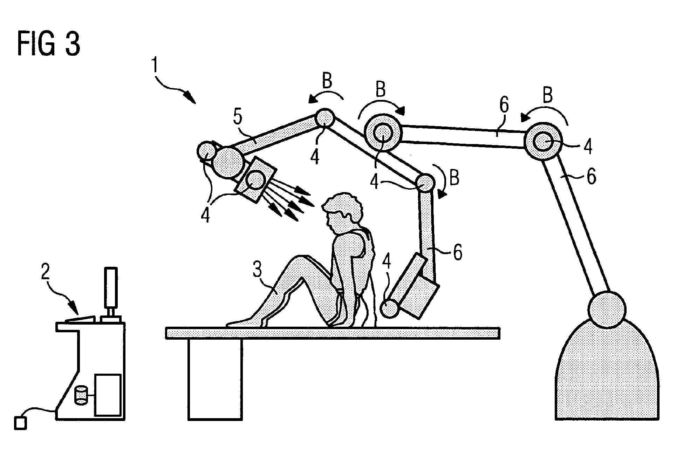

Fig. 3 zeigt ein erfindungsgemäßes Roboterarm-System mit verschiedenen Dreharmen 6, die verschiedene Drehbewegungen B ausführen können. Die Drehbewegungen B werden dabei in allen Richtungen ausgeführt, in dem die einzelnen Dreharme 6 durch Kugelgelenke verbunden sind, so dass das verfahrbare Teil in allen sechs Bewegungsrichtungen verfahrbar ist. Auch hier befinden sich Sensoren 4 an exponierter Stelle des verfahrbaren Teils 5 und messen die Entfernung zum Patienten 3. Diese Daten werden ebenfalls an das Regelgerät drahtgebunden oder drahtlos weitergeleitet. Das Regelgerät 2 steuert bzw. regelt die einzelnen Roboterarme 6 entsprechend den Abstandsdaten der Sensoren 4.

## Patentansprüche

1. Medizinisches Bildgebungssystem (1) einem Bildgebungsgerät und einer Röntgenröhre und mit einem relativ zum Patienten (3) verfahrbaren Teil (5), welches sich an einem Roboterarm-System mit verschiedenen Dreharmen (6), die verschiedene Drehbewegungen(B)ausführen können, befindet, wobei die Bewegung des verfahrbaren Teils (5) durch ein Regelgerät (2) gesteuert wird, welches die Bewegung stoppt oder verlangsamt, wenn das Teil (5) den Patienten oder ein anderes Objekt berührt oder sich annähert, **dadurch gekennzeichnet, dass**
mindestens ein Sensor (4) die Oberfläche des Patienten (3) oder des anderen Objekts erfasst und der mindestens eine Sensor (4) die Positionen einzelner Oberflächenbereiche des Patienten oder des anderen Objekts an das Regelgerät (2) übermittelt, das Regelgerät fortlaufend die Position des verfahrbaren Teils (5) durch einen zweiten Sensor (4, 8a, 8b) übermittelt bekommt, und das Regelgerät aus den Positionen die Abstände zwischen dem verfahrbaren Teil (5) und entsprechenden Oberflächenbereichen des Patienten (3) oder des anderen Objekts berechnet und die Geschwindigkeit und/oder die Verfahrrichtung, des verfahrbaren Teils (5) durch die Dreharme des Roboterarm-System entsprechend regelt, wobei die Sensoren (4, 8b) im Detektorgehäuse des Bildgebungsgeräts oder in bzw. an der Röntgenröhre oder anderen exponierten Bereichen des verfahrbaren Teils (5) integriert sind.

2. Bildgebungssystem nach einem der vorhergehenden Ansprüche, **dadurch gekennzeichnet, dass** der mindestens eine Sensor (4) ein Ultraschallsensor ist.

3. Bildgebungssystem nach einem der Ansprüche 1 bis 3, **dadurch gekennzeichnet, dass** der mindestens eine Sensor (4) ein optischer Sensor, insbesondere eine Kamera ist.

4. Bildgebungssystem nach einem der Ansprüche 1 bis 3, **dadurch gekennzeichnet, dass** der mindestens eine Sensor (4) ein elektromagnetischer Sensor, insbesondere ein Radargerät ist.

5. Bildgebungssystem nach einem der vorhergehenden Ansprüche, **dadurch gekennzeichnet, dass** der zweite Sensor (8a, 8b) ein elektromagnetischer Sensor ist, der aus einem ersten Sensorteil (8a) besteht, das ein elektromagnetisches Feld liefert und aus einem zweiten Sensorteil (8b), das am verfahrbaren Teil (5) angebracht ist und die Position des verfahrbaren Teils (5) im elektromagnetischen Feld detektiert.

6. Bildgebungssystem nach einem der vorhergehenden Ansprüche, **dadurch gekennzeichnet, dass** das Bildgebungssystem (1) ein Röntgensystem ist und das verfahrbare Teil (5) ein Röntgendetektor oder eine Röntgenröhre ist.

7. Bildgebungssystem nach einem der vorhergehenden Ansprüche, **dadurch gekennzeichnet, dass** mehrere Sensoren (4) an verschiedenen Stellen des verfahrbaren Teils (5) befestigt sind.

8. Bildgebungssystem nach einem der vorhergehenden Ansprüche, **dadurch gekennzeichnet, dass** das Koordinatensystem des Bildgebungssystems (1) und das der Sensoren (4) räumlich zueinander kalibriert sind.

9. Bildgebungssystem nach einem der vorhergehenden Ansprüche, **dadurch gekennzeichnet, dass** das Bildgebungssystem (1) dazu ausgelegt ist, aus der erfassten Oberfläche des Patienten (3) ein 3D-Modell des Patienten (3) zu erstellen und dieses mit dem Koordinatensystem des Bildgebungssystems (1) zu registrieren.

10. Kollisionsschutzverfahren für ein medizinisches Bildgebungssystem (1) mit einem um einen Patienten (3) verfahrbaren Teil (5), welches sich an einem Roboterarm-System mit verschiedenen Dreharmen (6), die verschiedene Drehbewegungen (B) ausführen können, befindet, insbesondere nach einem der vorhergehenden Ansprüche, zur Verhinderung einer Kollision des verfahrbaren Teils (5) mit einem anderen Objekt, wie z. B. dem Patienten (3), wobei die Bewegung des verfahrbaren Teils (5) in Abhängigkeit vom Abstand zum Patienten durch ein Regelgerät (2) regelbar ist, indem die Oberfläche des Patienten (3) oder eines anderen Objekts durch mindestens einen Sensor (4) erfasst wird und die Positionen einzelner Oberflächenbereiche des Patienten (3) oder des anderen Objekts an das Regelgerät (2) übermittelt werden, die Positionen des verfahrbaren Teils (5) durch einen zweiten Sensor (4, 8a, 8b) übermittelt werden und die Abstände zwischen dem verfahrbaren Teil (5) und den entsprechenden Oberflächenbereichen des Patienten (3) oder des anderen Objekts berechnet und das Regelgerät (2) aufgrund der berechneten Abstände zwischen dem verfahrbaren Teil (5) und der Oberfläche des Objekts die Geschwindigkeit und/oder die Verfahrrichtung des verfahrbaren Teils (5) durch die Dreharme des Roboterarm-System entsprechend regelt, wobei die Sensoren im Detektorgehäuse eines Bildgebungsgeräts oder in bzw. an einer Röntgenröhre oder anderen exponierten Bereichen des verfahrbaren Teils (5) integriert sind.

11. Kollisionsschutzverfahren nach Anspruch 10, **dadurch gekennzeichnet, dass** die Oberflächenerfassung durch einen optischen Sensor erfolgt und dass das Koordinatensystem des Bildgebungssystems (1) und das des optischen Sensors vor der Aufnahme von Bilddaten zueinander kalibriert werden.

## Claims

1. Medical imaging system (1) with an imaging device and an x-ray tube and with a moveable part (5) in respect of the patient (3), which is positioned on a robot arm system with different rotating arms (6), which can execute different rotary movements (B), with the movement of the moveable part (5) being controlled by a control device (2), which stops or slows down the movement, if the part (5) touches or approaches the patient or another object,
**characterised in that**
at least one sensor (4) detects the surface of the patient (3) or the other object and the at least one sensor (4) transmits the positions of individual surface areas of the patient or of the other object to the control device (2), the control device continuously obtains the position of the moveable part (5) in transmitted form by a second sensor (8a, 8b), and the control device calculates the distances between the moveable part (5) and corresponding surface areas of the patient (3) or another object from the positions and correspondingly controls the speed and/or the movement direction of the moveable part (5) by the rotating arms of the robot arm system, with the sensors (4, 8b) being integrated in the detector housing of the imaging device or in and/or on the x-ray tube or other exposed areas of the moveable part (5).

2. Imaging system according to one of the preceding claims,
**characterised in that**
the at least one sensor (4) is an ultrasound sensor.

3. Imaging system according to one of claims 1 - 3,
**characterised in that**
the at least one sensor (4) is an optical sensor, in particular a camera.

4. Imaging system according to one of claims 1-3,
**characterised in that**
the at least one sensor (4) is an electromagnetic sensor, in particular a radar device.

5. Imaging system according to one of the preceding claims,
**characterised in that**
the second sensor (8a, 8b) is an electromagnetic sensor, which comprises a first sensor part (8a), which supplies an electromagnetic field and a second sensor part (8b), which is attached to the moveable part (5) and detects the position of the moveable part (5) in the electromagnetic field.

6. Imaging system according to one of the preceding claims,
**characterised in that**
the imaging system (1) is an x-ray system and the moveable part (5) is an x-ray detector or an x-ray tube.

7. Imaging system according to one of the preceding claims,
**characterised in that**
a number of sensors (4) are affixed to different positions on the moveable part (5).

8. Imaging system according to one of the preceding claims,
**characterised in that**
the coordinate system of the imaging system (1) and that of the sensor (4) are spatially calibrated in respect of each other.

9. Imaging system according to one of the preceding claims,
**characterised in that**
the imaging system (1) is designed to create a 3D model of the patient (3) from the detected surface of the patient (3) and to register this with the coordinate system of the imaging system (1).

10. Anti-collision method for a medical imaging system (1) with a part (5) which can be moved about a patient (3), which is positioned on a robot arm system with different rotating arms (6), which can execute different rotary movements (B), in particular as claimed in one of the preceding claims, to prevent a collision of the moveable part (5) with another object, such as for instance the patient (3), with the movement of the moveable part (5) being controllable by a control device (2) as a function of the distance from the patient, in which control device the surface of the patient (3) or another object is detected by at least one sensor (4) and the positions of individual surface areas of the patient (3) or of the other object are transmitted to the control device (2), the positions of the moveable part (5) are transmitted by a second sensor (4, 8a, 8b) and the distances between the moveable part (5) and the corresponding surface areas of the patient (3) or of the other object are calculated and the control device (2) correspondingly controls the movement direction of the moveable part (5) by the rotating arms of the robot arm system on the basis of the calculated distances between the moveable part (5) and the surface of the object, with the sensors being integrated in the detector housing of an imaging device or in and/or on an x-ray tube or other exposed areas of the movable part (5).

11. Anti-collision method according to claim 10,
**characterised in that**
the surface detection is carried out by means of an optical sensor, and that the coordinate system of the imaging system (1) and that of the optical sensor are calibrated in respect of each other prior to recording the image data.

## Revendications

1. Système ( 1 ) d'imagerie médicale comprenant un appareil d'imagerie et un tube radiogène et une partie ( 5 ) qui peut être déplacée par rapport au patient ( 3 ) et qui se trouve sur un système de bras de robot ayant divers bras ( 6 ) tournants qui peuvent exécuter divers mouvements ( B ) de rotation, le mouvement de la partie ( 5 ) pouvant être déplacée étant commandé par un appareil ( 2 ) de réglage, qui arrête ou ralentit le mouvement lorsque la partie ( 5 ) touche le patient ou un autre objet ou s'en approche, **caractérisé en ce que**
au moins un capteur ( 4 ) détecte la surface du patient ( 3 ) ou de l'autre objet et le au moins un capteur ( 4 ) transmet les positions de divers parties superficielles du patient ou de l'autre objet à l'appareil de réglage, l'appareil de réglage reçoit en transmission par un deuxième capteur ( 4, 8a, 8b ) en continu la position de la partie ( 5 ) qui peut se déplacer et l'appareil de réglage calcule à partir des positions les distances entre la partie ( 5 ) qui peut se déplacer et des parties superficielles correspondantes du patient ( 3 ) ou de l'autre objet et règle en conséquence, par les bras tournants du système de bras de robot, la vitesse et/ou la direction de déplacement de la partie ( 5 ) qui peut se déplacer, les capteurs ( 4, 8b ) étant intégrés dans le boîtier de détecteur de l'appareil d'imagerie ou dans ou sur le tube radiogène ou d'autres parties exposées de la partie ( 5 ) pouvant se déplacer.

2. Système d'imagerie suivant l'une des revendications précédentes, **caractérisé en ce que** le au moins un capteur ( 4 ) est un capteur d'ultrason.

3. Système d'imagerie suivant l'une des revendications précédentes, **caractérisé en ce que** le au moins un capteur ( 4 ) est un capteur optique, notamment une caméra.

4. Système d'imagerie suivant l'une des revendications précédentes, **caractérisé en ce que** le au moins un capteur ( 4 ) est un capteur électromagnétique, notamment un appareil radar.

5. Système d'imagerie suivant l'une des revendications précédentes, **caractérisé en ce que** le deuxième capteur ( 8a, 8b ) est un capteur électromagnétique qui est constitué d'une première partie ( 8a ) de capteur qui fournit un champ électromagnétique et d'une deuxième partie ( 8b ) de capteur qui est mise sur la partie ( 5 ) pouvant se déplacer et qui détecte dans le champ électromagnétique la position de la partie ( 5 ) pouvant se déplacer.

6. Système d'imagerie suivant l'une des revendications précédentes, **caractérisé en ce que** le système ( 1 ) d'imagerie est un système de radiographie et la partie ( 5 ) qui peut se déplacer est un détecteur de rayon X ou un tube radiogène.

7. Système d'imagerie suivant l'une des revendications précédentes, **caractérisé en ce que** plusieurs capteurs ( 4 ) sont fixés en des points différents de la partie ( 5 ) pouvant se déplacer.

8. Système d'imagerie suivant l'une des revendications précédentes, **caractérisé en ce que** le système de coordonnées du système ( 1 ) d'imagerie et celui des capteurs ( 4 ) sont étalonnés l'un par rapport à l'autre dans l'espace.

9. Système d'imagerie suivant l'une des revendications précédentes, **caractérisé en ce que** le système ( 1 ) d'imagerie est tel qu'à partir de la surface détectée du patient ( 3 ) on peut établir un modèle en 3D du patient ( 3 ) et l'enregistrer avec le système de coordonnées du système ( 1 ) d'imagerie.

10. Procédé de protection à l'encontre d'une collision pour un système ( 1 ) d'imagerie médical, comprenant une partie ( 5 ) qui peut se déplacer autour d'un patient ( 3 ) et qui se trouve sur un système de bras de robot ayant des divers bras ( 6 ) tournants pouvant exécuter divers mouvements ( B ) de rotation, notamment suivant l'une des revendications précédentes, pour empêcher une collision de la partie ( 5 ) qui peut se déplacer avec un autre objet, comme par exemple le patient ( 3 ), le mouvement de la partie ( 5 ) qui peut se déplacer pouvant être réglé par un appareil ( 2 ) de réglage en fonction de la distance au patient en détectant la surface du patient ou d'un autre objet par au moins un capteur ( 4 ) et en transmettant les positions des diverses parties superficielles du patient ( 3 ) ou de l'autre objet à l'appareil ( 2 ) de réglage, les positions de la partie ( 5 ) qui peuvent se déplacer étant transmises par un deuxième capteur ( 4, 8a, 8b ) et les distances entre la partie ( 5 ) qui peut se déplacer et les parties superficielles correspondantes du patient ( 3 ) ou de l'autre objet étant calculées et l'appareil ( 2 ) de réglage réglant en conséquence, par les bras tournants du système de bras de robot, la vitesse et/ou la direction de déplacement de la partie ( 5 ) qui peut se déplacer sur la base des distances calculées entre la partie ( 5 ) qui peut se déplacer à la surface de l'objet, les capteurs étant intégrés dans le boîtier de détecteur d'un appareil d'imagerie ou dans ou sur un tube radiogène ou d'autres parties exposées de la partie ( 5 ) qui peut se déplacer.

11. Procédé de protection vis-à-vis d'une collision suivant la revendication 10, **caractérisé en ce que** l'on effectue la détection de surface par un capteur optique et **en ce que** l'on étalonne l'un par rapport à l'autre le système de coordonnées du système ( 1 ) d'imagerie et celui du capteur optique avant d'enregistrer des données d'images.
